# DEMANDE DE BREVET EUROPEEN

(11) **EP 1 068 860 A1**
(43) Date de publication de la demande: **17.01.2001**
(21) Numéro de dépôt: 00401884.2
(22) Date de dépôt: 30.06.2000
(51) Int. Cl.: A61K 7/13

(54) **Composition tinctoriale contenant du 1-acétoxy-naphtalène, deux bases d'oxydation et un coupleur**

(30) Priorité: 12.07.1999 FR 9909011
(71) Demandeur: L'OREAL, 75008 Paris (FR)
(72) Inventeur: Audousset, Marie-Pascale, 92600 Asnières (FR)
(74) Mandataire: Miszputen, Laurent

(57) **Abrégé**

L'invention a pour objet une composition pour la teinture d'oxydation des fibres kératiniques contenant du 1-acétoxy-2-méthyl-naphtalène à titre de premier coupleur, au moins deux bases d'oxydation différentes l'une de l'autre, et au moins un deuxième coupleur ; ainsi que le procédé de teinture d'oxydation mettant en oeuvre cette composition.

## Description

L'invention a pour objet une composition pour la teinture d'oxydation des fibres kératiniques contenant du 1-acétoxy-2-méthyl-naphtalène à titre de premier coupleur, au moins deux bases d'oxydation différentes l'une de l'autre, et au moins un deuxième coupleur ; ainsi que le procédé de teinture d'oxydation mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bis-phénylalkylènediamines ou bien encore des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, en particulier dans le brevet US 5,344 463 d'utiliser à titre de coupleur, certains dérivés 2-substitués du naphtol. Cependant, ces composés présentent une mauvaise stabilité au stockage. Dans les brevets US 5 529 583 et US 5 672 180, il a ensuite été proposé d'utiliser à titre de coupleur, d'autres dérivés substitués du 2-méthyl naphtol tel que le 1-acétoxy-2-méthyl-naphtalène afin de remédier à ce problème de stabilité au stockage. Cependant, bien qu'étant stable au stockage, le 1-acétoxy-2-méthyl-naphtalène utilisé à titre de coupleur ne donne pas entièrement satisfaction, car il conduit avec la plupart des bases d'oxydation à des colorations sélectives, et ne présentant pas une résistance suffisante aux différents traitements que peuvent subir les cheveux.

Il a été proposé également dans la demande de brevet EP-A-920 853 des compositions de teinture d'oxydation prêtes à l'emploi contenant au moins un dérivé substitué du 2-méthyl naphtol comme coupleur et au moins une para-aminophénol comme base d'oxydation, un oxydant, un tensio-actif, de l'eau ; lesdites compositions ayant un HLB cumulatif allant jusqu'à 2,5. Ces compositions conduisent à des colorations dont les propriétés ne sont pas encore pleinement satisfaisantes.

Or, la Demanderesse vient maintenant de découvrir, de façon totalement inattendue et surprenante, que l'association du 1-acétoxy-2-méthyl-naphtalène, avec au moins deux bases d'oxydation dont une choisie parmi les para-aminophénols substitués et au moins un coupleur additionnel permettait d'obtenir des colorations puissantes et peu sélectives, présentant de plus des propriétés de résistance améliorées vis à vis des diverses agressions que peuvent subir les cheveux (shampooings, lumière, intempéries, ondulations permanentes, transpiration, frottements, etc...).

La Demanderesse a également découvert, de façon totalement inattendue et surprenante, que l'association du 1-acétoxy-2-méthyl-naphtalène, avec au moins deux bases d'oxydation et au moins un coupleur additionnel convenablement sélectionné permettait également d'obtenir des colorations puissantes et peu sélectives, présentant de plus des propriétés de résistance améliorées vis à vis des diverses agressions que peuvent subir les cheveux.

Ces découvertes sont à la base de la présente invention.

L'invention a pour premier objet une composition pour la teinture d'oxydation des fibres kératiniques, et en particulier des fibres kératiniques humaines telles que les cheveux, caractérisée par le fait qu'elle contient, dans un milieu approprié pour la teinture au moins :
- du 1-acétoxy-2-méthyl-naphtalène, à titre de premier coupleur ;
- au moins une première base d'oxydation choisie parmi les para-aminophénols substitués ;
- au moins une deuxième base d'oxydation choisie parmi les paraphénylènediamines, les bases doubles et les bases d'oxydation hétérocycliques ; - et au moins un deuxième coupleur.

Les coupleurs additionnels pouvant être utilisés dans les compositions tinctoriales de l'invention sont choisis de préférence parmi les métaphénylènediamines, les méta-aminophénols, les métadiphénols, , les mono ou polyhydroxy naphtalènes, les coupleurs hétérocycliques.

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture :
- du 1-acétoxy-2-méthyl-naphtalène, à titre de premier coupleur ;
- au moins deux bases d'oxydation ;
- et au moins un deuxième coupleur choisi parmi les méta-phénylènediamines substituées, le méta-aminophénol , les méta-diphénols substitués , les mono- ou polyhydroxy naphtalènes, les coupleurs hétérocycliques.

Comme indiqué précédemment, les compositions tinctoriale conformes à l'invention conduisent à des colorations puissantes, peu sélectives et qui présentent de plus d'excellentes propriétés de résistance vis à vis de l'action des différents agents extérieurs (lumière, intempéries, lavage, ondulation permanente, transpiration, frottements).

Parmi les paraphénylènediamines utilisables à titre de base d'oxydation dans les composition tinctoriales conformes à l'invention, on peut notamment citer les composés de formule (I) suivante, et leurs sels d'addition avec un acide : 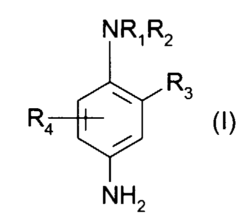 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés de formule (II) suivante, et leurs sels d'addition avec un acide : 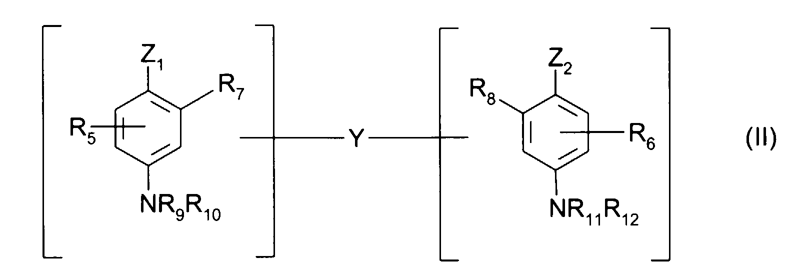 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthyl-aminophényl) tétraméthylènediamine, la N,N'-bis-(éthyl) N,N'-bis-(4'-amino, 3'-méthylphényl)éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de base d'oxydation dans les compositions tinctoriales conformes à l'invention, on peut notamment citer :
- le para-aminophénol de structure suivante : 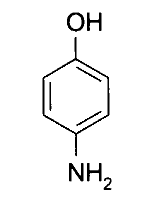
- les para-aminophénols substitués de formule (III) suivante : 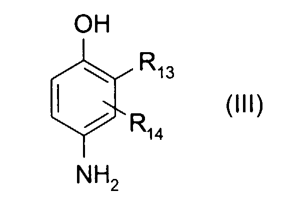 dans laquelle :
   - R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
   - R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₁₃ ou R₁₄ est différent d'un atome d'hydrogène ; ainsi que leurs sels d'addition avec un acide.

Parmi les para-aminophénols substitués de formule (III) ci-dessus, on peut plus particulièrement citer le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957, DE 4 234 885, DE 19 64 30 59 et les demandes de brevet WO 94/08969, WO 94/08970, FR-A-2 733 749 et DE 195 43 988 comme le 4,5-diamino 1-méthyl pyrazole, le 4,5-diamino-1-hydroxyethyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

L'ensemble des bases d'oxydation représente de préférence de 0,0005 à 12 % en poids environ du poids total de la composition tinctoriale conforme à l'invention, et encore plus préférentiellement de 0,005 à 6 % en poids environ de ce poids.

Parmi les méta-phénylènediamines utilisables à titre de deuxième coupleur dans les compositions tinctoriales conformes à l'invention, on peut notamment citer
- la méta-phénylènediamine de formule : 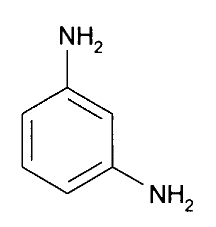 et les méta-phénylènediamines substituées de formule (IV) suivante : 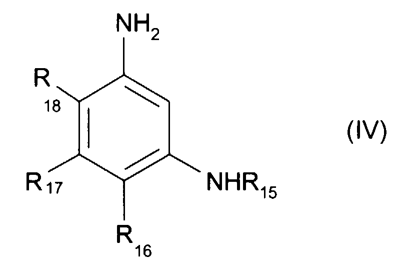 dans laquelle :
   - R₁₅ représente un atome d'hydrogène, un radical alkyle en C₁-C₄, monohydroxyalkyle en C₁-C₄ ou polyhydroxyalkyle en C₂-C₄ ;
   - R₁₆ et R₁₇, identiques ou différents, représentent un atome d'hydrogène, un radical alkyle en C₁-C₄ ou hydroxyalkoxy en C₁-C₄ ;
   - R₁₈ représente un atome d'hydrogène, un radical alcoxy en C₁-C₄, aminoalcoxy en C₁-C₄, monohydroxyalcoxy en C₁-C₄, polyhydroxyalcoxy en C₂-C₄ ou un radical 2,4-diaminophénoxyalkoxy, étant entendu que l'un des radicaux R₁₅, R₁₆, R₁₇ et R₁₈ est différent d'hydrogène ; ainsi que leurs sels d'addition avec un acide.

Parmi les méta-phénylènediamines substituées de formule (IV) ci-dessus, on peut plus particulièrement citer le 2,4-diamino phénoxyéthanol, le 2,4-diamino 1-éthoxybenzène, le 2-amino 4-N-(β-hydroxyéthyl)amino anisole, le 3,5-diamino 1-éthyl 2-méthoxybenzène, le 3,5-diamino 2-méthoxy 1-méthyl benzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2-amino 4-N-(β-hydroxyéthyl) amino 1-méthoxy benzène, et leurs sels d'addition avec un acide.

Parmi les méta-aminophénols utilisés à titre de coupleur dans les compositions tinctoriales conformes à l'invention on peut plus particulièrement citer
- le méta-aminophénol de structure : 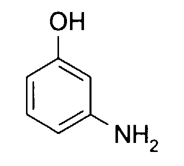
- les méta-aminophénols susbstitués de formule (V) suivante, et leurs sels d'addition avec un acide : 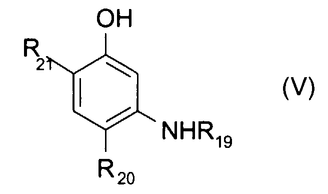 dans laquelle :
   - R₁₉ représente un atome d'hydrogène, un radical alkyle en C₁-C₄, monohydroxyalkyle en C₁-C₄ ou polyhydroxyalkyle en C₂-C₄,
   - R₂₀ représente un atome d'hydrogène, un radical alkyle en C₁-C₄, alcoxy en C₁-C₄ ou un atome d'halogène choisi parmi le chlore, le brome ou le fluor,
   - R₂₁ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄.

Parmi les méta-aminophénols substitués de formule (V) ci-dessus, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

Parmi les méta-diphénols utilisables à titre de deuxième coupleur dans les compositions tinctoriales conformes à l'invention, on peut notamment citer :
- la résorcine de structure suivante : 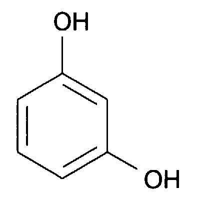
- les méta-diphénols substitués de formule (VI) suivante : 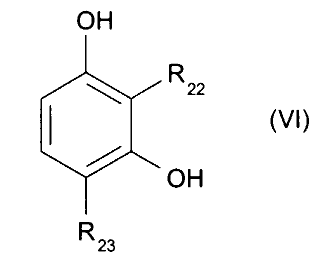 dans laquelle :
   - R₂₂ et R₂₃, identiques ou différents, représente un atome d'hydrogène, un radical alkyle en C₁-C₄ ou un atome d'halogène choisi parmi le chlore, le brome ou le fluor ; étant entendu qu'au moins un des radicaux R₂₂ et R₂₃ est différent d'un atome d'hydrogène ; ainsi que leurs sels d'addition avec un acide :

Parmi les méta-diphénols substitués de formule (VI) ci-dessus, on peut plus particulièrement citer le 2-méthyl 1,3-dihydroxy benzène, le 4-chloro 1,3-dihydroxy benzène, le 2-chloro 1,3-dihydroxybenzène, et leurs sels d'addition avec un acide.

Parmi les mono- ou poly-hydroxynaphtalènes on peut citer plus particulièrement : l'α-naphtol, le 1,7-dihydroxy naphtalène, le 2,7-dihydroxy naphtalène, le 2,5-dihydroxy naphtalène, le 2,3-dihydroxy naphtalène, le 1-hydroxy 2-méthyl naphtalène, l'acide 1-hydroxy 4-naphtalène sulfonique et leurs sels d'addition avec un acide.

Parmi les coupleurs hétérocycliques, on peut citer par exemple les dérivés indoliques, les dérivés indoliniques, les dérivés pyridiniques et les pyrazolones, le sésamol et ses dérivés et leurs sels d'addition avec un acide et plus particulièrement le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, le 1-H 3-méthyl pyrazole 5-one, le 1-phényl 3-méthyl pyrazole 5-one, le sésamol, la 2,6-diaminopyridine, la 2-amino-3-hydroxypyridine, la 3,5-diamino-2,6-diméthoxypyridine, le 1-β-hydroxyéthylamino-3,4-méthylènedioxybenzène,le 1-amino -3,4-méthylènedioxybenzène et leurs sels d'addition avec un acide.

Le ou les deuxièmes coupleurs représentent de préférence de 0,0001 à 10 % en poids environ du poids total de la composition tinctoriale et encore plus préférentiellement de 0,005 à 5 % en poids environ de ce poids.

Le 1-acétoxy-2-méthyl-naphtalène représente de préférence de 0,0005 à 12% en poids environ du poids total de la composition tinctoriale, et encore plus préférentiellement de 0,005 à 6 % en poids environ de ce poids.

D'une manière générale, les sels d'addition avec un acide utilisables dans le cadre de l'invention (bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 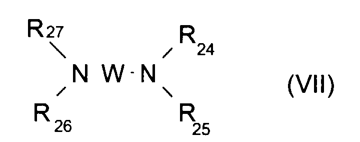 dans laquelle W est un reste propylène éventuellement substitué par un groupement hydroxyle ou un radical alkyle en C₁-C₆; R₂₄, R₂₅, R₂₆ et R₂₇, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples suivants sont destinés à illustrer l'invention sans pour autant présenter un caractère limitatif.

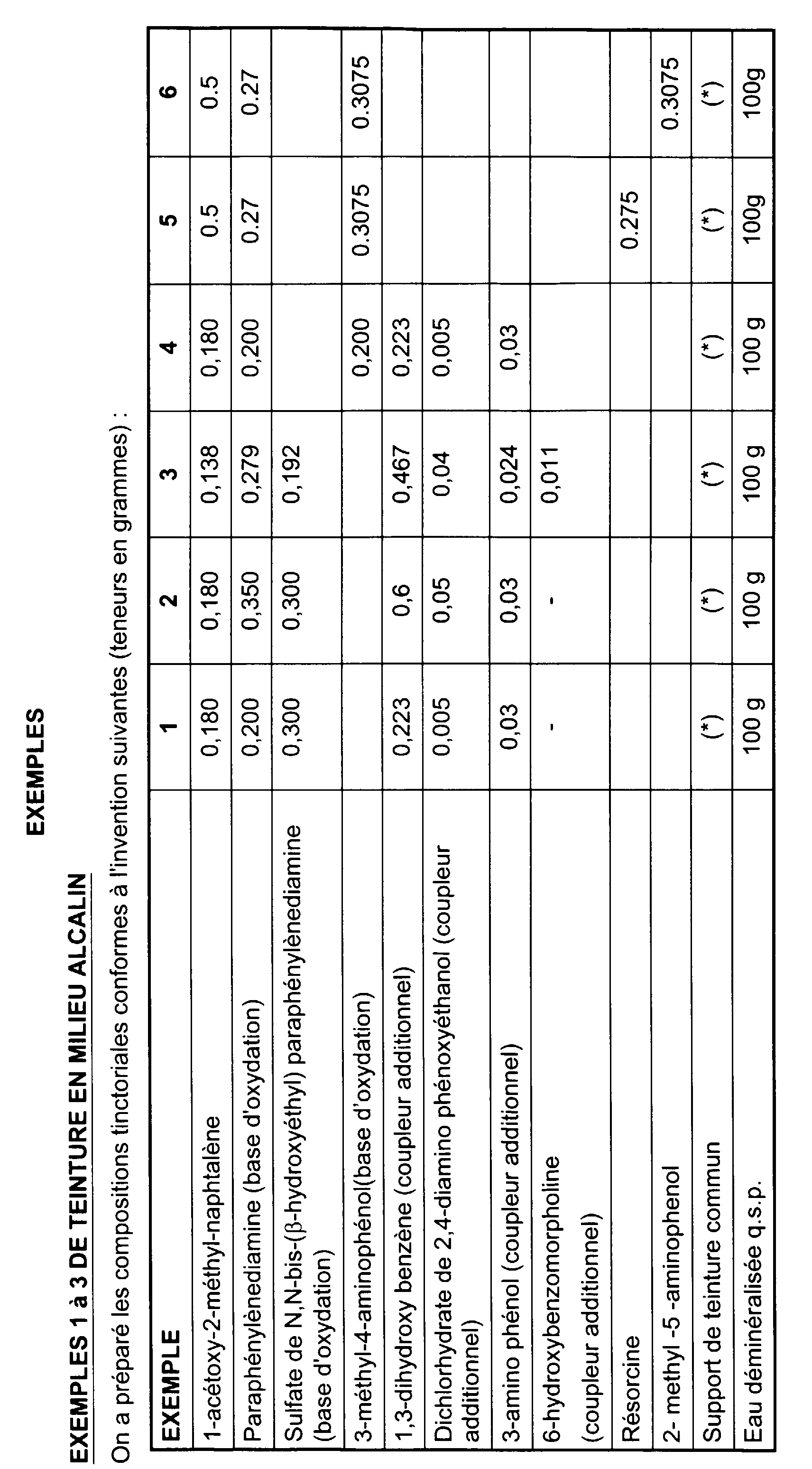

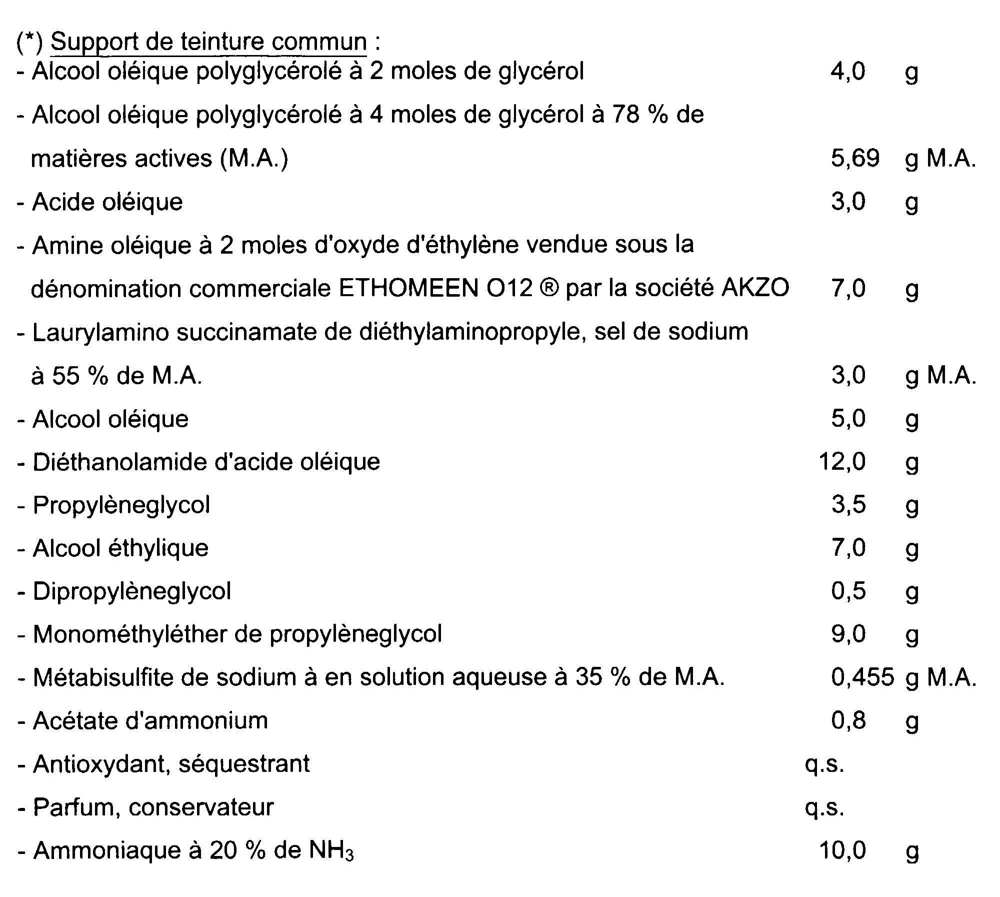

Au moment de l'emploi, on a mélangé poids pour poids chacune des compositions tinctoriales ci-dessus avec une solution de peroxyde d'hydrogène à 20 volumes (6 % en poids) de pH 3. Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées. Les nuances obtenues figurent dans le tableau ci-après :

| **EXEMPLE** | **Nuance obtenue** |
|---|---|
| **1** | Blond naturel doré cendré |
| **2** | Blond foncé cendré irisé |
| **3** | Blond foncé naturel cendré |
| **4** | blond naturel doré |
| **5** | Blond naturel doré irisé |
| **6** | Blond irisé cendré |

## Revendications

1. Composition pour la teinture d'oxydation des fibres kératiniques, et en particulier des fibres kératiniques humaines telles que les cheveux, caractérisée par le fait qu'elle contient, dans un milieu approprié pour la teinture :
- du 1-acétoxy-2-méthyl-naphtalène, à titre de premier coupleur ;
- au moins une première base d'oxydation choisie parmi les para-aminophénols substitués ;
- au moins une deuxième base d'oxydation additionnelle choisie parmi les paraphénylènediamines, les bases doubles et les bases d'oxydation hétérocycliques ;
- et au moins un deuxième coupleur.

2. Composition selon la revendication 1 caractérisée par le fait que le deuxième coupleur est choisi parmi les méta-phénylènediamines , les méta-aminophénols , les méta-diphénols, les mono- ou polyhydroxy naphtalènes, les coupleurs hétérocycliques

3. Composition pour la teinture d'oxydation des fibres kératiniques, et en particulier des fibres kératiniques humaines telles que les cheveux, caractérisée par le fait qu'elle contient, dans un milieu approprié pour la teinture :
- du 1-acétoxy-2-méthyl-naphtalène, à titre de premier coupleur ;
- au moins deux bases d'oxydation ;
- et au moins un deuxième coupleur choisi parmi les métaphénylènediamines substituées, le méta-aminophénol , les méta-diphénols substitués, les mono ou polyhydroxynaphtalènes, les coupleurs hétérocycliques.

4. Composition selon la revendication 3 caractérisée par le fait que les bases d'oxydation sont choisies parmi les paraphénylènediamines, les paraaminophénols, les bases doubles et les bases d'oxydation hétérocycliques ;

5. Composition selon les revendications 1 ou 4, caractérisée par le fait que les paraphénylènediamines sont choisies parmi les composés de formule (I) suivante, et leurs sels d'addition avec un acide : 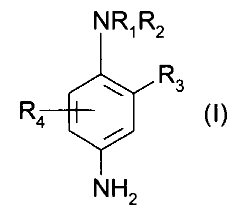 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

6. Composition selon la revendication 5, caractérisée par le fait que les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

7. Composition selon les revendications 1 ou 4, caractérisée par le fait que les bases doubles sont choisies parmi les composés de formule (II) suivante, et leurs sels d'addition avec un acide : 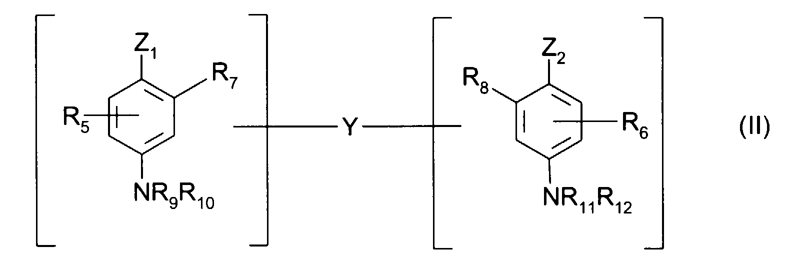 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄; étant entendu que les composés de formule (II) ne comportent qu'un seul bras de liaison Y par molécule.

8. Composition selon la revendication 7, caractérisée par le fait que les bases doubles de formules (II) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

9. Composition selon les revendications 1 ou 4, caractérisée par le fait que les bases d'oxydation hétérocycliques sont choisies parmi les dérivés pyridiniques, les dérivés pyrimidiniques, les dérivés pyrazoliques, le sésamol et ses dérivés et leurs sels d'addition avec un acide.

10. Composition selon la revendication 9, caractérisée par le fait que les dérivés pyridiniques sont choisis parmi la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide ; que les dérivés pyrimidiniques sont choisis parmi la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques ; et que les dérivés pyrazoliques sont choisis le 4,5-diamino 1-méthyl pyrazole, le 4,5-diamino-1-hydroxyethyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

11. Composition selon la revendication 10, caractérisée par le fait que les dérivés pyrazolo-pyrimidiniques sont choisis parmi la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

12. Composition selon l'une quelconque des revendications 1, 2, 4 à 11, où les para-aminophénols sont choisis dans le groupe constitué par :
- le para-aminophénol de structure suivante : 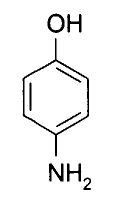
- les para-aminophénols substitués de formule (III) suivante : 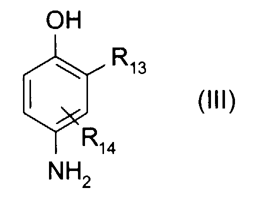 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₁₃ ou R₁₄ est différent d'un atome d'hydrogène ; ainsi que leurs sels d'addition avec un acide.

13. Composition selon la revendication 12, où les para-aminophénols substitués de formule (III) sont choisis parmi le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

14. Composition selon l'une quelconque des revendications 1 à 13, caractérisée par le fait que l'ensemble des bases d'oxydation représente de 0,0005 à 12 % en poids du poids total de la composition tinctoriale.

15. Composition selon la revendication 14, caractérisée par le fait que l'ensemble des bases d'oxydation représente de 0,005 à 6 % en poids du poids total de la composition tinctoriale.

16. Composition selon l'une quelconque des revendications 2 à 15, où les méta-phénylènediamines sont choisies dans le groupe constitué par :
- la méta-phénylènediamine de formule : 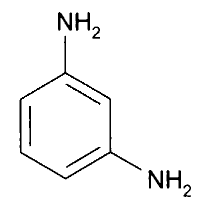
- les méta-phénylènediamines substituées de formule (IV) suivante : 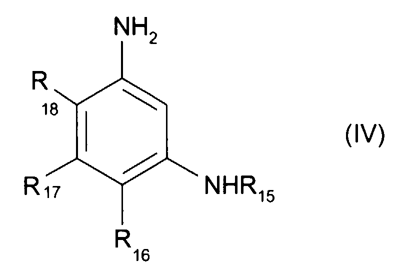 dans laquelle :
- R₁₅ représente un atome d'hydrogène, un radical alkyle en C₁-C₄, monohydroxyalkyle en C₁-C₄ ou polyhydroxyalkyle en C₂-C₄ ;
- R₁₆ et R₁₇, identiques ou différents, représentent un atome d'hydrogène, un radical alkyle en C₁-C₄ ou hydroxyalkoxy en C₁-C₄ ;
- R₁₈ représente un atome d'hydrogène, un radical alcoxy en C₁-C₄, aminoalcoxy en C₁-C₄, monohydroxyalcoxy en C₁-C₄, polyhydroxyalcoxy en C₂-C₄ ou un radical 2,4-diaminophénoxyalkoxy, étant entendu que l'un des radicaux R₁₅, R₁₆, R₁₇ et R₁₈ est différent d'hydrogène ; ainsi que leurs sels d'addition avec un acide :

17. Composition selon la revendication 16 où les méta-phénylènediamines substituées de formule (IV) sont choisies parmi le 2,4-diamino phénoxyéthanol, le 2,4-diamino 1-éthoxybenzène, le 2-amino 4-N-(β-hydroxyéthyl)amino anisole, le 3,5-diamino 1-éthyl 2-méthoxybenzène, le 3,5-diamino 2-méthoxy 1-méthyl benzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2-amino 4-N-(β-hydroxyéthyl) amino 1-méthoxy benzène, et leurs sels d'addition avec un acide.

18. Composition selon l'une quelconque des revendications 2 à 15, où les méta-aminophénols sont choisis dans le groupe constitué par :
- le méta-aminophénol de structure : 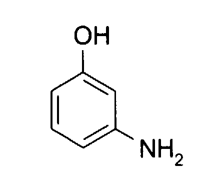
- les méta-aminophénols susbstitués de formule (V) suivante, et leurs sels d'addition avec un acide : 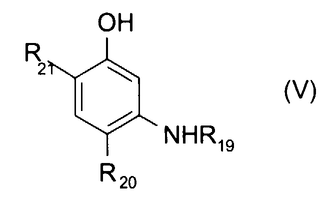 dans laquelle :
- R₁₉ représente un atome d'hydrogène, un radical alkyle en C₁-C₄, monohydroxyalkyle en C₁-C₄ ou polyhydroxyalkyle en C₂-C₄,
- R₂₀ représente un atome d'hydrogène, un radical alkyle en C₁-C₄, alcoxy en C₁-C₄ ou un atome d'halogène choisi parmi le chlore, le brome ou le fluor,
- R₂₁ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄.

19. Composition selon la revendication 18 où les méta-aminophénols substitués sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

20. Composition selon l'une quelconque des revendications 2 à 15, où les méta-diphénols sont choisis dans le groupe constitué par :
- la résorcine de structure suivante : 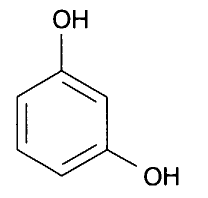
- les méta-diphénols substitués de formule (VI) suivante : 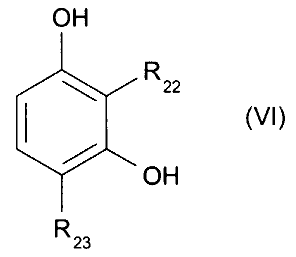 dans laquelle :
- R₂₂ et R₂₃, identiques ou différents, représente un atome d'hydrogène, un radical alkyle en C₁-C₄ ou un atome d'halogène choisi parmi le chlore, le brome ou le fluor ; étant entendu qu'au moins un des radicaux R₂₂ et R₂₃ est différent d'un atome d'hydrogène ; ainsi que leurs sels d'addition avec un acide :

21. Composition selon la revendication 20, où les méta-diphénols substitués de formule (VI) sont choisis parmi le 2-méthyl 1,3-dihydroxy benzène, le 4-chloro 1,3-dihydroxy benzène, le 2-chloro 1,3-dihydroxybenzène, et leurs sels d'addition avec un acide.

22. Composition selon l'une quelconque des revendications 2 à 15 où les mono- ou poly-hydroxynaphtalènes sont choisis parmi l'α-naphtol, le 1,7-dihydroxy naphtalène, le 2,7-dihydroxy naphtalène, le 2,5-dihydroxy naphtalène, le 2,3-dihydroxy naphtalène, le 1-hydroxy 2-méthyl naphtalène, l'acide 1-hydroxy 4-naphtalène sulfonique et leurs sels d'addition avec un acide.

23. Composition selon l'une quelconque des revendications 2 à 15, caractérisée par le fait que les coupleurs hétérocycliques sont choisis parmi les dérivés indoliques, les dérivés indoliniques, les dérivés pyridiniques et les pyrazolones, le sésamol et ses dérivés et leurs sels d'addition avec un acide

24. Composition selon la revendications 23, caractérisée par le fait que les coupleurs hétérocycliques sont choisis parmi le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, le 1-H 3-méthyl pyrazole 5-one, le 1-phényl 3-méthyl pyrazole 5-one, le sésamol, la 2,6-diaminopyridine ;la 2-amino-3-hydroxypyridine,la 3,5-diamino-2,6-diméthoxypyridine, le 1-β-hydroxyéthylamino-3,4-méthylènedioxybenzène,le 1-amino-3,4-méthylènedioxybenzène et leurs sels d'addition avec un acide.

25. Composition selon l'une quelconque des revendications 1 à 24, caractérisée par le fait que le ou les deuxièmes coupleurs représentent de 0,0001 à 10 % en poids du poids total de la composition tinctoriale.

26. Composition selon la revendication 25, caractérisée par le fait que le ou les deuxièmes coupleurs représentent de 0,005 à 5 % en poids du poids total de la composition tinctoriale.

27. Composition selon l'une quelconque des revendications 1 à 24, caractérisée par le fait que le 1-acétoxy-2-méthyl-naphtalène représente de 0,0005 à 12% en poids du poids total de la composition tinctoriale.

28. Composition selon la revendication 27, caractérisée par le fait que le 1-acétoxy-2-méthyl-naphtalène représente de 0,005 à 6% en poids du poids total de la composition tinctoriale.

29. Composition selon l'une quelconque des revendications 1 à 28, caractérisée par le fait que les sels d'addition avec un acide sont choisis parmi les chlorhydrates, les bromhydrates, les sulfates, les citrates, les succinates, les tartrates, les lactates et les acétates.

30. Procédé de teinture d'oxydation des fibres kératiniques et en particulier des fibres kératiniques humaines telles que les cheveux caractérisé par le fait qu'on applique sur lesdites fibres au moins une composition tinctoriale telle que définie à l'une quelconque des revendications 1 à 29, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

31. Procédé selon la revendication 30, caractérisé par le fait que l'agent oxydant est choisi parmi le peroxyde d'hydrogène, le peroxyde d'urée, les bromates de métaux alcalins, les persels, et les enzymes.

32. Dispositif à plusieurs compartiments, ou "kit" de teinture à plusieurs compartiments, dont un premier compartiment renferme une composition tinctoriale telle que définie à l'une quelconque des revendications 1 à 29 et un second compartiment renferme une composition oxydante.
